## Europäisches Patentamt

## European Patent Office

## Office européen des brevets

(11) Veröffentlichungsnummer: **0 428 927 B1**

(12) **EUROPÄISCHE PATENTSCHRIFT**

(45) Veröffentlichungstag der Patentschrift: **07.06.95**

(51) Int. Cl.6: **A61M 1/16**

(21) Anmeldenummer: **90121077.3**

(22) Anmeldetag: **03.11.90**

(54) **Verfahren zur Bestimmung von Hämodialyse-Parametern während der Hämodialyse.**

(30) Priorität: **21.11.89 DE 3938662**

(43) Veröffentlichungstag der Anmeldung:
**29.05.91 Patentblatt 91/22**

(45) Bekanntmachung des Hinweises auf die
Patenterteilung:
**07.06.95 Patentblatt 95/23**

(84) Benannte Vertragsstaaten:
**DE ES FR GB IT SE**

(56) Entgegenhaltungen:
**EP-A- 0 272 414**
**US-A- 4 508 622**

(73) Patentinhaber: **Fresenius AG**
**Gluckensteinweg 5**
**D-61350 Bad Homburg (DE)**

(72) Erfinder: **Polaschegg, Hans-Dietrich, Dr.**
**Grünwiesenweg 9**
**W-6370 Oberursel 4 (DE)**

(74) Vertreter: **Dr. Fuchs, Dr. Luderschmidt Dr.**
**Mehler, Dipl.-Ing. Weiss Patentanwälte**
**Postfach 46 60**
**D-65036 Wiesbaden (DE)**

Rank Xerox (UK) Business Services
(3.10/3.09/3.3.3)

**Beschreibung**

Die Erfindung bezieht sich auf ein Verfahren zur in-vivo-Bestimmung von Parametern der Hämodialyse unter Verwendung einer Vorrichtung zur Einstellung der Ionen-Konzentration der Dialysierflüssigkeit und einer Meßvorrichtung.

Die Hämodialyse wird seit einer Vielzahl von Jahren erfolgreich zur Behandlung von Patienten verwendet und hat sich weltweit bewährt. Obwohl die Hämodialyse einen hohen Entwicklungsstand erreicht hat, ergeben sich dennoch einige wichtige Gesichtspunkte, aufgrund derer Weiterentwicklungen der Hämodialyseverfahren notwendig sind. Dabei spielt insbesondere der finanzielle Aufwand zur langzeitigen Behandlung von chronisch kranken Patienten eine wichtige Rolle. Sowohl durch die Kostenentwicklung des Gesundheitssystems in den industriealisierten Ländern als auch hinsichtlich der finanziellen Problematik der Entwicklungsländer ist es nötig, den Aufwand für die Hämodialyse weiter zu senken, um die Behandlung einer weiter ansteigenden Zahl von Patienten zu ermöglichen. Der Kostenfaktor der Hämodialyse hängt entscheidend davon ab, daß das Verfahren optimal durchgeführt werden kann, da zum einen die Behandlungszeit bei einer optimalen Durchführung des Verfahrens verkürzt werden kann und da andererseits die zusätzlichen Pflegeaufwendungen für einen Patienten, welcher durch eine optimale Hämodialyse gut versorgt ist, geringer sind, da der Patient weniger morbide ist und daher weniger Pflege braucht.

Im Rahmen der NCCLS wurde in den USA die Morbidität eines großen Patientenkollektivs in Abhängigkeit von der Dialysedosis untersucht. Dabei stellte sich heraus, daß die Morbidität auf einen niedrigen, konstanten Wert absinkt, wenn der Wert für K t/V von 0,8 auf größer oder gleich 1 ansteigt.

Dabei ist K die Clearance, t die Behandlungszeit und V das Gesamtkörperwasser des Patienten. Es ist deshalb anzustreben, die Behandlung jeweils so zu steuern, daß K t/V für jede Behandlung 1 wird.

Bei der Optimierung der Hämodialyse ist es somit, um einen Anstieg der Morbidität zu vermeiden, unumgänglich, das obengenannte Verhältnis K t/V entweder exakt zu bestimmen oder aus Sicherheitsgründen die Dialysezeit zu erhöhen. Letzteres führt, abgesehen von möglichen gesundheitlichen Risiken zu einer erheblichen Kostenbelastung. Zur Optimierung der Hämodialyse ist es relativ einfach möglich, das Gesamtkörperwasser V des Patienten zu bestimmen, da dieses über einen längeren Zeitraum bekannt ist. Das Gesamtkörperwasser läßt sich aus dem Körpergewicht, dem Alter und dem Geschlecht sowie dem geschätzten Fettanteil des Patienten abschätzen. Es läßt sich auch mit Hilfe von Harnstoffmessungen und dem Harnstoffmodell errechnen. Zusätzlich kann auch die Gesamtkörperimpedanzmessung zur Bestimmung herangezogen werden. Weiterhin ist es relativ einfach, die Behandlungszeit t für die jeweilige Behandlung des Patienten zu bestimmen.

Demgegenüber sind aus dem Stand der Technik keine in-vivo-anwendbaren Verfahren bekannt, die Clearance des Hämodialysators exakt zu bestimmen. Es wird deshalb üblicherweise von den Angaben des Herstellers ausgegangen, welche jedoch beträchtliche Fehler aufweisen. Der Hersteller prüft zum einen nicht jeden einzelnen Dialysator auf dessen Clearance, vielmehr wird vom Hersteller ein bestimmter Clearance-Bereich garantiert, wodurch jedoch nicht ausgeschlossen ist, daß einige Dialysatoren aus diesem Bereich fallen.

Ein weiterer Grund für Änderungen der Clearance besteht darin, daß insbesondere in den USA aus Kosten- und aus Biokompatibilitätsgründen Dialysatoren aufbereitet und wiederverwendet werden.

Ein weiterer Grund, warum die Herstellerangaben über die Clearance nicht exakt stimmen besteht darin, daß die Herstellerangaben auf Messungen in wässriger Lösung beruhen, während die bei der Behandlung eines Patienten relevante Clearance sich auf den wässerigen Anteil des Blutes bezieht. Der Fluß dieses wässrigen Anteils wird aus dem gesamten Blutfluß und dem Hämatokrit des Patienten errechnet, welcher zwar in der Regel bekannt ist, sich aber über die Dauer der Hämodialyse als Folge der Ultrafiltration ändert.

Zusätzlich ist zu berücksichtigen, daß der Blutfluß nur annäherungweise bekannt ist, da er üblicherweise von der Förderrate, d.h. der Umdrehungsgeschwindigkeit einer üblicherweise eingesetzten peristaltischen Blutpumpe und dem Schlauchdurchmesser des Systems abhängt. Da der Schlauchdurchmesser nur mit einer Genauigkeit von +/- 5 % bekannt ist, und da weiterhin der Pumpquerschnitt durch den Ansaugunterdruck verringert werden kann, ergeben sich auch hierbei erhebliche Toleranzen.

Aus alledem folgt, daß es aus dem Stand der Technik nicht möglich ist, die Clearance in-vivo zu messen und daß die bisher bekannten Berechnungsmethoden erhebliche Ungenauigkeiten aufweisen.

Zur Lösung dieser Schwierigkeiten ist es aus dem Stand der Technik bekannt, in-vivo Harnstoffmessungen durchzuführen, da die relevante Clearance des Dialysators die Harnstoff-Clearance ist, da die obengenannte Berechnungsformel auf dem Harnstoffmodell beruht. Zur Harnstoffmessung müssen Proben genommen und im Labor untersucht werden, wobei es zu erheblichen Verzögerungen zwischen der Probenentnahme und der Mitteilung des Meßergebnisses kommt. Eine Steuerung der Dialysebehandlung ist somit nicht möglich. Weiterhin ist dieses Untersuchungsverfahren sehr kostenintensiv. In der Regel wird deshalb die Behandlung des Patienten aufgrund von Schätzwerten und einem Sicherheitszuschlag durchgeführt, wobei lediglich in größeren Zeitabständen, beispielsweise einmal im Monat mittels Harnstoffmessungen eine Qualitätsprüfung durchgeführt wird.

Es ist aus dem Stand der Technik weiterhin bekannt, daß die Clearance für Natrium-Chlorid-Ionen der Harnstoff-Clearance gleich ist. Da die Ionen-Konzentration und damit die Leitfähigkeit der Dialysierflüssigkeit und des Blutes im wesentlichen durch Na- und Cl-Ionen hervorgerufen werden, ist es möglich, die Clearance durch eine Leitfähigkeitsmessung zu bestimmen. Bei einem bekannten Verfahren wird zur Bestimmung der Clearance die Konzentration auf der Bluteingangsseite des Hämodialysators vorgegeben und auf der Dialysierflüssigkeit-Eingangsseite auf Null gesetzt oder umgekehrt. Dieses Verfahren weist jedoch den Nachteil auf, daß es während der Dialyse einerseits nicht möglich ist, die Eingangskonzentration auf der Dialysierflüssigkeit-Eingangsseite auf Null zu setzen und andererseits die Eingangskonzentration des Blutes nicht bekannt ist. Deshalb eignet sich dieses Verfahren nicht für eine in-vivo-Steuerung der Hämodialyse.

Der Erfindung liegt die Aufgabe zugrunde, ein Verfahren der eingangs genannten Art zu schaffen, mit Hilfe dessen Parameter der Hämodialyse, insbesondere die Clearance des Dialysators bestimmt werden kann und welches in einfacher und betriebssicherer Weise bei geringem apparatetechnischem Aufwand eine in-vivo-Messung der Parameter ermöglicht.

Erfindungsgemäß wird die Aufgabe dadurch gelöst, daß mittels der Meßvorrichtung der Elektrolyttransfer der Dialysierflüssigkeit bei zwei unterschiedlichen, vorgegebenen Dialysierflüssigkeits-Ionenkonzentrationen gemessen wird und daß hieraus die Dialysance bestimmt wird.

Das erfindungsgemäße Verfahren zeichnet sich durch eine Reihe erheblicher Vorteile aus. Erfindungsgemäß ist es nämlich möglich, mittels einer einfach aufgebauten und betriebssicher zu handhabenden Einrichtung, welche beispielsweise aus der DE-A-32 23 051 beschrieben ist, die entsprechenden Meßwerte zu ermitteln. Dabei ist es möglich, die Na-Ionenkonzentration bzw. die Leitfähigkeit stromauf bzw. stromab des Dialysators in der Dialysierflüssigkeit zu messen.

Als besonders günstig erweist es sich, daß erfindungsgemäß die Messung bei zwei unterschiedlichen, vorgegebenen Ionen-Konzentrationen erfolgt, welche in entsprechender Weise eingestellt werden können. Es ist dabei möglich, diese Konzentrationen auf physiologisch vertretbare Werte einzustellen, ohne daß hierdurch die Hämodialyse beeinträchtigt oder gestört würde. Ein Vorteil der Erfindung liegt darin, daß die beiden unterschiedlichen Konzentrationswerte, welche eingestellt werden, innerhalb eines sehr kurzen Zeitraums von wenigen Minuten eingestellt werden können, so daß davon ausgegangen werden kann, daß sich die Blutkonzentration Cbi und die Dialysance D im betrachteten Intervall nicht ändern.

Da erfindungsgemäß die zulaufende Dialysierflüssigkeit bereits eine bestimmte Eingangskonzentration aufweist, ist es möglich, die Gleichung für die Dialysance wie folgt aufzustellen, wobei hinsichtlich der Berechnungsgrundlagen auf Sargent, J.A., Gotch, F.A., : Principles and biophysics of dialysis, in: Replacement of Renal Function by Dialysis, W. Drukker, F.M.Parsons, J.F.Maher (Hrsg), Nijhoff, Den Haag 1983 verwiesen wird.

$$D = Qb \cdot \frac{Cbi - Cbo}{Cbi - Cdi} \quad (1)$$

Aus Gründen der Massenbilanz gilt:

$$Qb \cdot (Cbi - Cbo) = -Qd \cdot (Cdi - Cdo) \quad (2)$$

Aus den beiden obengenannten Gleichungen (1) und (2) folgt:

$$D = -Qd \cdot \frac{Cdi - Cdo}{Cbi - Cdi} \quad (3)$$

Dabei sind:

Cbi: Bluteingangskonzentration
Cbo: Blutausgangskonzentration
Qb : Blutfluß
D : Dialysance
Cdi: Dialysierflüssigkeit-Eingangskonzentration
Cdo: Dialysierflüssigkeit-Ausgangskonzentration
Qd : Dialysierflüssigkeits-Fluß

Da Qd und Cdi vorgegeben sind und der Wert Cdo gemessen wird, sind in dem obengenannten Gleichungssystem lediglich D und Cbi unbekannt. Es liegen erfindungsgemäß somit zwei Gleichungen mit zwei Unbekannten vor, welche sich wie folgt lösen lassen:

$$D = Qd \cdot \frac{(Cdi1 - Cdo1) - (Cdi2 - Cdo2)}{Cdi1 - Cdi2} \quad (4)$$

Mittels der Gleichung (4) ist die Dialysance unter Verwendung des erfindungsgemäßen Verfahrens bestimmbar. Ihr Zahlenwert ist identisch mit der gesuchten Clearance des Hämodialysators.

Die in der Gleichung (4) angegebenen Indices 1 und 2 beziehen sich jeweils auf die erste bzw. zweite Einstellung der Dialysierflüssigkeitskonzentration.

In einer besonders günstigen Ausgestaltung der Erfindung ist vorgesehen, daß die beiden Messungen im kurzen zeitlichen Abstand zueinander erfolgen. Diese Maßnahme stellt sicher, daß sich die übrigen Parameter erfahrungsgemäß nicht weiter ändern und somit als konstant angesehen werden können.

In einer besonders vorteilhaften Weiterbildung der Erfindung ist vorgesehen, daß die Natrium-Ionen-Konzentration der Dialysierflüssigkeit gemessen wird. Alternativ dazu ist es auch möglich, die temperaturkorrigierte Leitfähigkeit der Dialysierflüssigkeit zu bestimmen, wobei dies bevorzugterweise unter Verwendung eines Leitfähigkeitssensors erfolgt.

Die erfindungsgemäße Auswertung der Meßwerte erfolgt vorteilhafterweise gemäß der oben angegebenen Gleichung (4), welche den Vorteil aufweist, daß die Blutkonzentration in der Gleichung nicht als Zahlenwert in Erscheinung tritt.

Das erfindungsgemäße Verfahren gilt zusätzlich zur reinen Dialyse auch für eine Dialyse mit Ultrafiltration, es ist auch möglich, die Messung selbst bei ausgeschalteter Ultrafiltration vorzunehmen. Sofern das Ergebnis des erfindungsgemäßen Bestimmungsverfahrens zur Vorherbestimmung der notwendigen Dialysezeit herangezogen wird, kann auf jegliche Korrektur des Meßergebnisses verzichtet werden, d.h., es kann direkt mit dem durch die Gleichung (4) ermittelten Resultat, in dem der Einfluß der Ultrafiltration implizit enthalten ist, gearbeitet werden.

Unter Zugrundelegung des erfindungsgemäßen Verfahrens ist es möglich, eine Veränderung des Blutvolumens des Patienten festzustellen. Eine derartige Veränderung des Blutvolumens führt bekanntlicherweise zu einem Anstieg des Hämatrokrit und einer Abnahme des wässrigen Anteils des Blutes. Da sich die Clearance bzw. die Dialysance auf den wässrigen Anteil des Blutes bezieht, sinkt diese, wenn der wässrige Anteil des Blutes zurückgeht. Als Grund hierfür ist anzusehen, daß die obengenannten Erläuterungen streng genommen nur für wässrige Lösungen gelten, während die Gleichung (4) auch für Blut gilt, da die Blutkonzentration der Gleichung eliminiert ist.

Die Bluteingangskonzentration Cbi läßt sich aus der obigen Ableitung nur für wässrige Lösungen bestimmen. Bei Blut ist es hingegen erforderlich, den Gibbs-Donnan-Koeffizienten zu berücksichtigen. Dabei wird hier unter Blutkonzentration die Konzentration in der wässrigen Phase des Blutes verstanden, welche z.B. mittels eines Ionometers bestimmt wird. Die Bluteingangskonzentration Cbi läßt sich wie folgt darstellen:

$$Cbi = \frac{Cdi'}{\alpha} \quad (5),$$

4

wobei ($\alpha$) der sogenannte Gibbs-Donnan-Koeffizient ist und Cdi' ist die Konzentration in der Dialysierflüssigkeit in einem Vergleichsversuch unter Gleichgewichtsbedingungen,

Für diesen gilt nach Sargent und Gotch:

$$\alpha = 1 - 0,0074 \, Ctp \qquad (6)$$

wobei Ctp die Plasmaproteinkonzentration gemessen in (g/dl) ist.

Ist die Plasmaproteinkonzentration bzw. der Wert $\alpha$ bekannt, so kann die Blutkonzentration unter Berücksichtigung der Gleichungen (1 bis 6) wie folgt angegeben werden:

$$Cbi' \times \alpha = Cdi - \frac{Qd}{D} (Cdi - Cdo) \qquad (7)$$

Sofern erfindungsgemäß der Quotient aus gemessener Dialysance (Clearance) und Blutfluß verfolgt wird, kann daraus auf die Veränderung des Blutvolumens geschlossen werden. Hierbei ist allerdings Voraussetzung, daß die Transporteigenschaften der Dialysemembran unverändert bleiben, d.h., daß es nicht zu einer teilweise Blockade der Membran kommt. Derartige Störungen der Hämodialyse sind jedoch mittels geeigneter Detektoren feststellbar.

Weiterhin ist es erfindungsgemäß günstig, daß mittels des Verfahrens auf Veränderungen der Transporteigenschaften der Membran geschlossen werden kann, sofern andere Methoden zur Bestimmung der Veränderung des Blutvolumens vorhanden sind. Aus dem Vergleich der damit berechneten Änderungen der Clearance mit der gemessenen Änderung können somit Veränderungen der Transporteigenschaft der Membran bestimmt werden.

Um mit Hilfe des erfindungsgemäßen Verfahrens auch die Möglichkeit zu haben, die Abhängigkeit der Paramter von dem Dialysierflüssigkeitsfluß zu bestimmen, ist es in einer Weiterbildung der Erfindung vorgesehen, auch den Dialysierflüssigkeltsfluß auf unterschiedliche Werte zu variieren, so daß entsprechend der Gleichung (4) die Dialysance in Abhängigkeit vom pialysierflüssigkeitsfluß bestimmt werden kann. Es läßt sich somit jener Dialysierflüssigkeitsfluß ermitteln, bei welchem z.B. ein bestimmter Prozentsatz der Dialysance bei einem vorgegebenen pialysierflüssigkeitsfluß erreicht wird, beispielsweise 1000 ml/min oder Qd = 2 Qb. Es ist somit unter Verwendung des erfindungsgemäßen Verfahrens möglich, einen Dialysierflüssigkeitsfluß einzustellen, bei welchem sich die Behandlung des Patienten besonders kostengünstig durchführen läßt.

Die Erfindung ist nicht auf die gezeigten Ausführungsbeispiele und Anwendungsmöglichkeiten beschränkt, vielmehr ergeben sich im Rahmen der Erfindung vielfältige Weiterentwicklungen, insbesondere hinsichtlich der Variation der entsprechenden Parameter.

## Patentansprüche

1. Verfahren zur in-vivo-Bestimmung von Parametern der Hämodialyse unter Verwendung einer Vorrichtung zur Einstellung der Ionenkonzentration der Dialysierflüssigkeit und einer Meßvorrichtung, dadurch gekennzeichnet, daß mittels der Meßvorrichtung der Elektrolyttransfer der Dialysierflüssigkeit bei zwei unterschiedlichen, vorgegebenen Dialysierflüssigkeits-Ionenkonzentrationen gemessen wird und daß hieraus die Dialysance bestimmt wird.

2. Verfahren nach Anspruch 1, dadurch gekennzeichnet, daß die beiden Messungen in kurzem zeitlichem Abstand erfolgen.

3. Verfahren nach Anspruch 1 oder 2, dadurch gekennzeichnet, daß die Na-Konzentration der Dialysierflüssigkeit gemessen wird.

4. Verfahren nach einem der Ansprüche 1 oder 2, dadurch gekennzeichnet, daß die temperaturkorrigierte Leitfähigkeit der Dialysierflüssigkeit gemessen wird.

5. Verfahren nach einem der Ansprüche 1 - 4, dadurch gekennzeichnet, daß die Messung unter Verwendung von Leitfähigkeitssensoren erfolgt.

6. Verfahren nach einem der Ansprüche 1 - 5, dadurch gekennzeichnet, daß zur Bestimmung der Dialysance die Differenz zwischen den Differenzen der Dialysierflüssigkeits-Ionenkonzentration an der Eingangsseite und der Ausgangsseite des Dialysators zum Zeitpunkt der ersten und der zweiten Messung bestimmt wird, durch die Differenz der Dialysierflüssigkeits-Ionenkonzentration an der Eingangsseite zum Zeitpunkt der ersten Messung und der zweiten Messung geteilt wird und mit dem Dialysierflüssigkeits-Fluß multipliziert wird.

7. Verfahren nach einem der Ansprüche 1 - 6, dadurch gekennzeichnet, daß die Clearance des Dialysators aus der ermittelten Dialysance festgestellt wird.

8. Verfahren nach einem der Ansprüche 1 - 7, dadurch gekennzeichnet, daß Veränderungen des wässrigen Anteils des Blutes durch Messung von Veränderungen der Clearance und/oder der Dialysance ermittelt werden.

9. Verfahren nach einem der Ansprüche 1 - 7, dadurch gekennzeichnet, daß Veränderungen des Blutvolumens bestimmt und mit Änderungen der Clearance und/oder Dialysance verglichen werden und daß daraus Veränderungen der Transporteigenschaften der Membrane bestimmt werden.

10. Verfahren nach einem der Ansprüche 1 - 9, dadurch gekennzeichnet, daß der Dialysierflüssigkeits-Fluß auf zwei unterschiedliche Werte eingestellt wird und daß die Dialysance in Abhängigkeit vom Dialysierflüssigkeits-Fluß bestimmt wird.

## Claims

1. A method for the in-vivo determination of hemodialysis parameters using an apparatus for adjusting the ion concentration of the dialysis fluid and a measuring device, characterized in that the electrolyte transfer of said dialysis fluid is measured by means of said measuring device at two different predetermined dialysis fluid ion concentrations and that the dialysance is determined on the basis thereof.

2. A method according to claim 1, characterized in that the two measurements are carried out at a short time interval.

3. A method according to claim 1 or 2, characterized in that the Ny concentration of said dialysis fluid is measured.

4. A method according to any of claims 1 or 2, characterized in that the temperature-corrected conductivity of said dialysis fluid is measured.

5. A method according to any of claims 1 to 4, characterized in that the measurement is carried out by using conductivity sensors.

6. A method according to any of claims 1 to 5, characterized in that for the determination of said dialysance the difference is determined between the differences of said dialysis fluid ion concentration at the inlet side and the outlet side of the dialyzer at the time of the first and second measurements, and is divided by the difference of said dialysis fluid ion concentration at said inlet side at the time of said first and said second measurements and multiplied by the dialysis fluid flow.

7. A method according to any of claims 1 to 6, characterized in that the clearance of said dialyzer is determined on the basis of said determined dialysance.

8. A method according to any of claims 1 to 7, characterized in that changes in the aqueous portion of blood are determined by measuring changed in said clearance and/or said dialysance.

9. A method according to any of claims 1 to 7, characterized in that changes in the blood volume are determined and compared with changes in said clearance and/or said dialysance and that changes in the transportation properties of the membrane are determined on the basis thereof.

10. A method according to any of claims 1 to 9, characterized in that said dialysis fluid flow is adjusted to two different values and that said dialysance is determined in dependance upon said dialysis fluid flow.

**Revendications**

1. Procédé de détermination in-vivo de paramètres de l'hémodialyse en utilisant un appareil de réglage de la concentration en ions du liquide de dialyse et un instrument de mesure, procédé caractérisé en ce que l'on mesure avec l'instrument de mesure le transfert d'électrolytes du liquide de dialyse à deux concentrations en ions du liquide différentes, prédonnées, et on en détermine la dialysance.

2. Procédé selon la revendication 1, caractérisé en ce que l'on effectue les deux mesures à un court intervalle de temps.

3. Procédé selon la revendication 1 ou 2, caractérisé en ce que l'on mesure la concentration en Na du liquide de dialyse.

4. Procédé selon une des revendications 1 ou 2, caractérisé en ce que l'on mesure la conductibilité du liquide de dialyse corrigée en fonction de la température.

5. Procédé selon l'une des revendications 1 à 4, caractérisé en ce que l'on effectue la mesure avec des capteurs de conductibilité.

6. Procédé selon l'une des revendications 1 à 5, caractérisé en ce que, pour déterminer la dialysance, on détermine la différence entre les différences de la concentration en ions du liquide de dialyse à l'entrée et à la sortie du dialyseur aux moments de la première et de la seconde mesure, on divise le résultat par la différence de la concentration en ions du liquide à l'entrée aux moments de la première et de la seconde mesure, et on multiplie par le flux du liquide de dialyse.

7. Procédé selon l'une des revendications 1 à 6, caractérisé en ce que l'on établit la clairance du dialyseur d'après la dialysance déterminée.

8. Procédé selon l'une des revendications 1 à 7, caractérisé en ce que l'on détermine des modifications de la teneur en eau du sang par la mesure de modifications de la clairance et/ou de la dialysance.

9. Procédé selon l'une des revendications 1 à 7, caractérisé en ce que l'on détermine des modifications du volume sanguin, que l'on compare avec des modifications de la clairance et/ou de la dialysance, et on en détermine des changements des propriétés de transport de la membrane.

10. Procédé selon l'une des revendications 1 à 9, caractérisé en ce que l'on règle le flux du liquide de dialyse à deux valeurs différentes et on détermine la dialysance en fonction du flux du liquide de dialyse.